# EUROPEAN PATENT SPECIFICATION

(11) **EP 2 412 680 B1**
(45) Date of publication and mention of the grant of the patent: **28.03.2018**
(21) Application number: 11006028.2
(22) Date of filing: 22.07.2011
(51) Int. Cl.: C02F 3/08, C02F 3/10

(54) **improved biological reactor of the fixed biomass type**
Verbesserter biologischer Festbiomassenreaktor
Réacteur biologique amélioré pour le type de biomasse fixe

(30) Priority: 28.07.2010 IT MI20101389
(43) Date of publication of application: 01.02.2012
(73) Proprietor: Gattoni, Marino, 28021 Borgomanero (NO) (IT); Geuna, Enrico, 20131 Milano (IT)
(72) Inventor: Gattoni, Marino, 28021 Borgomanero (NO) (IT); Geuna, Enrico, 20131 Milano (IT)
(74) Representative: Lecce, Giovanni

(56) References cited:
- DE-U1- 29 802 186
- GB-A- 508 881
- US-A- 4 330 408

## Description

The present invention relates to an improved biological reactor with fixed biomass.

More in particular, the present invention relates to a biological reactor of the adhering or fixed biomass type used in the operation for depurating industrial and/or civil liquid waste and in all applications wherein the culture of microorganisms is required for microbiological processes suitable for preparing chemical and pharmaceutical substances.

As is known, purification plants perform a series of treatments aimed at removing the pollutants present in wastewaters, for example of household, industrial, agricultural origin or of other type.

There are multiple types of reactors and among them, the biological reactors with fixed biomass are generally used if due to waste waters with a mainly organic matrix with a high specific load, the use of activated sludge biological reactors is not productive and/or easy.

Said reactors traditionally comprise contactors defined by elements or discs rotating relative to a horizontal axis, made of a polymeric material and immersed in basins by 40, 60% of the diameter thereof; the rotation is imparted by means of a traditional gearmotor.

The contactor structure is such as to allow, in association with the hydrostatic thrust exerted by a gas, the rotation of the same contactor around the axis thereof.

Following the rotation of the contactor, the microorganisms present in the waste waters or in the slurry passing in the basins begin to colonize the surface of the same and to multiply up to covering the entire surface with a layer consisting of biomass or biofilm and a concentration up to forty times higher than that characterising the suspended biomass systems; in the case of reaction conducted with the rotors partly immersed, besides the exchange due to the air/gas blown from the bottom, with the rotation of the contactor the slurry film comes into contact also with the atmosphere, drops and absorbs oxygen.

In this way, the growth of the biomass fixed to the contactor, the contact of the biomass with the slurry and the aeration of the same are allowed; since they are bacteria colonies adhering to the support, a natural stripping occurs and the contactor mixing action keeps the solids detached in a suspension up until the exit from the reaction basin.

However, the contactors of the traditional biological reactors exhibit some drawbacks related to mechanical stresses, in particular the twisting moment on the masses involved, which cause mechanical breakage.

A further drawback is related to the proliferation of undesired microorganisms which form a gelatinous mass that does not allow a sufficient oxygen intake to the biofilm or biomass, thus preventing the growth of aerobic microorganisms useful for purifying the waste waters.

In order to obviate such drawbacks, an improved type of contactor has been developed (object of patent EP0853067) defined by a plurality of channels arranged parallel to the rotation axis, with the concave portion oriented downwards so as to allow the filling thereof with the air blown from the bottom of the basin and favour the contactor rotation by the effect of Archimedes' principle. The same contactor further comprises a plurality of channels arranged on the side opposite the rotation axis and with the concavity oriented upwards; in this way, the air blown does not fill the cavities and there is no braking action on the cylinder.

As a consequence of what described above, the exchange surface between the biomass and the slurry is improved with a consequent improved efficiency of the reactor comprising the contactor.

However, such channels have a specific surface the average value whereof is of 150 m2/m3.

Larger specific surfaces imply a higher density of the channels with a reduction of the spaces comprised thereinbetween; as a consequence thereof, there is the risk of a weakening of the structure and the forming of preferential channels which cause an uneven development of the biomass or biofilm layer.

DE29802186 discloses a floating apparatus for the treatment of waste water that is made to rotate around an horizontal axis by a flow of forced air, in which the oxygen for the biological reactions comes directly from environmental air. US 4330408 discloses an apparatus for aqueous waste treatment by means of a rotating biological contactor partially submerged in waste and carrying oxidative microorganism. The object of the present invention is to obviate the drawbacks of the traditional equipment mentioned above.

More in particular, the object of the present invention is to provide an improved biological reactor with fixed biomass such as to ensure an exchange surface between biomass and slurry that is larger than the contactors of known reactors, so as to increase and optimise the performance and the efficiency of the same reactor.

A further object of the present invention is to increase the specific surface in a flexible and easy manner and thus, the system performance without weakening the contactor structure.

A further object of the present invention is to provide a biological reactor suitable for ensuring a high level of safety and reliability over time and also such as to be easily and inexpensively constructed.

These and other objects are achieved by the improved biological reactor as claimed in claim 1. The construction and functional features of the improved biological reactor with fixed biomass of the present invention shall be better understood from the following detailed description, wherein reference is made to the annexed drawing table showing a preferred and nonlimiting embodiment thereof, wherein:

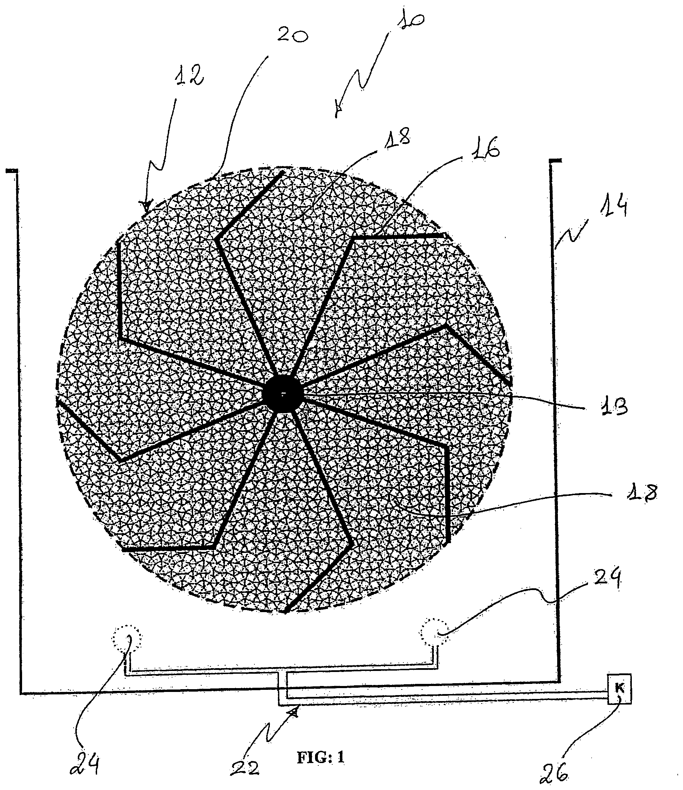

figure 1 shows a schematic cross section view of the improved biological reactor with fixed biomass of the invention.

With reference to said figure, the improved biological reactor with fixed biomass of the invention, globally indicated with reference numeral 10, comprises a biological contactor 12 rotating relative to a horizontal axis 13 and arranged within a tank or basin 14 partly or entirely filled with liquid or waste water or other solution to be treated, with said basin that may be open or closed according to whether the reactor operation is in an aerobic or anaerobic environment.

The biological contactor 12 comprises a plurality of walls 16 that, starting preferably but not exclusively from the centre of the contactor, develop radially, in a continuous or discontinuous manner, according to a rectilinear and basically radial direction, shaped with one or more bends for defining a dome structure; the radial arrangement of walls 16 may be more or less regular according to the specific process requirements.

In an alternative embodiment, the same walls 16 may have a curvilinear development.

Moreover, walls 16 develop longitudinally in a continuous manner or stretch-wise by the entire length of contactor 12.

The walls thus arranged define angular sectors wherein a plurality of filling bodies 18 of any shape and size are inserted.

Walls 16 and the filling bodies 18 are arranged so as to fill the entire volume of the biological contactor 12.

The filling bodies 18 have a specific surface comprised between 200 and 500 m2/m3 and preferably equal to 280 m2/m3.

The assembly defined by walls 16 and by the filling bodies 18 is wound in a containment cage defined by a metal net 20, preferably but not exclusively with large mesh so as to allow an easy exit of the bacterial spoils developed during the process.

The contactor so defined is partly or fully immersed in basin 14 on the bottom whereof there is arranged a plant 22 for diffusing the air provided with one or more diffusers 24 and supplied by a compressor 26; basin 14 may be of the open or closed type according to the type of aerobic or anaerobic process.

Walls 16, along with the filling bodies 18, cover and fill the entire contactor structure allowing the air blown by plant 22 to penetrate up to the innermost parts of the same contactor so as to favour, at the same time, the rotational thrust and an optimal exchange between microorganisms, gas and liquid on the entire surface thereof.

The provision of walls 16 and of the filling bodies 18 allows an advantageous and optimal air blowing without opposing the rotation of the same contactor in any way.

Walls 16 and the filling bodies 18 of contactor 12 may be made with different types of material, such as for example polymeric, composite or the like and they may have different geometrical section profiles.

Moreover, the surface of walls 16 and of the filling bodies 18 may be corrugated so as to make the biomass or biofilm development easier and increase the exchanges between gas and liquid with a specific surface larger than that of the traditional devices.

As can be noticed from the above, the advantages achieved by the reactor of the invention are clear.

Thanks to the provision of the walls and of the filling elements, the improved biological reactor with fixed biomass of the present invention ensures an exchange surface between biomass and liquid or nourishing solution that is considerably larger than the contactors of the known reactors, and as a consequence it increases and optimises the performance and the yield of the same reactor.

A further advantage is the fact that the number of filling bodies inserted in the space comprised between two walls may be changed according to the specific process requirements; in this way, the specific surface may be changed even during the process so as to adjust the progress of biomass development, consumption of the organic contents or of the dedicated production and the exchange between air and liquid in an optimal manner.

A further advantage is that the operating features of the reactor of the invention may be easily and quickly changed allowing the control of the associated costs.

## Claims

1. A biological reactor (10) of the adhering or fixed biomass type, for depurating industrial or civil liquid waste and/or culturing microorganism for microbiological processes in preparing chemical and pharmaceutical substances, comprising:
- a tank or basin (14) suitable for containing a nourishing substance or waste water;
- a biological contactor (12) partly or completely immersed in said tank (14) and rotatable relative to a horizontal axis (13);
- a compressor (26) and one or more diffusers (24) arranged on the bottom of said tank (14), suitable for generating and blowing a flow of air or gas for rotating said contactor (12);
said contactor (12) comprising a plurality of concave sectors defined by walls (16) with a shape having one or more sharp angles arranged within the contactor (12), longitudinally extended continuously or in portions by the entire lenght of the contactor (12) and filling bodies (18) arranged within said sectors, filling the entire volume of the biological contactor (12) and suitable for increasing the exchange surface between microorganism, gas and liquid;
containment means enclosing said sectors and filling bodies (18) and allowing the passage of liquids.

2. The biological reactor according to claim 1, **characterised in that** said sectors ara defined by walls (16) develops radially basically starting from the axis of rotation (13).

3. The biological reactor according to claim 1 or 2, **characterised in that** said walls (16) are developed according to a rectilinear and basically radial direction and are shaped with one or more bends for defining some domes.

4. The biological reactor according to claim 2, **characterised in that** said walls (16) have a curvilinear development.

5. The biological reactor according to claim 1, **characterised in that** the filling bodies (18) have a specific surface comprised between 200 and 500 m2/m3.

6. The biological reactor according to claim 1 to 5, **characterised in that** said filling bodies (18) have a specific surface equal to 280 m2/m3.

7. The biological reactor according to one or more of the previous claims, **characterized in that** said containment means is a cage defined by a metal net (20) whose meshes ara suitable for allowing an easy exit of bacterial spoils developed during the process.

## Patentansprüche

1. Biologischer Reaktor (10) vom anhaftenden oder fixierten Biomassetyp zur Reinigung von industriellen oder zivilen flüssigen Abfällen und/oder Kultivierungsmikroorganismen für mikrobiologische Prozesse bei der Herstellung chemischer und pharmazeutischer Substanzen, umfassend:
- einen Tank oder ein Becken (14), geeignet zur Aufnahme von Nährstoffen oder Abwasser;
- einen biologischen Kontaktgeber (12), der teilweise oder vollständig in den Tank (14) eingetaucht und relativ zu einer horizontalen Achse (13) drehbar ist;
- einen Kompressor (26) und einen oder mehrere Diffusoren (24), die am Boden des Tanks (14) angeordnet sind und geeignet sind, einen Luft- oder Gasstrom zum Drehen des Kontaktgebers (12) zu erzeugen und zu blasen;
wobei der Kontaktgeber (12) eine Vielzahl von konkaven Sektoren aufweist, die durch Wände (16) mit einer Form mit einem oder mehreren spitzen Winkeln definiert sind, die innerhalb des Kontaktgebers (12) angeordnet sind und sich kontinuierlich oder abschnittsweise über die gesamte Länge des Kontaktgebers (12) longitudinal erstrecken und Füllkörper (18), die innerhalb der Sektoren angeordnet sind, das gesamte Volumen des biologischen Kontaktgebers (12) ausfüllen und geeignet sind, die Austauschfläche zwischen Mikroorganismen, Gas und Flüssigkeit zu vergrößern;
wobei Einschließungsmittel die Sektoren und Füllkörper (18) umschließen und den Durchgang von Flüssigkeiten ermöglichen.

2. Biologischer Reaktor nach Anspruch 1, **dadurch gekennzeichnet, dass** die Winkelsektoren durch Wände (16) definiert sind, die radial im Wesentlichen ausgehend von der Drehachse (13) radial verlaufen.

3. Biologischer Reaktor nach Anspruch 1 oder 2, **dadurch gekennzeichnet, dass** die Wände (16) in einer geradlinigen und im Wesentlichen radialen Richtung ausgebildet sind und mit einer oder mehreren Krümmungen zum Definieren einiger Kuppeln geformt sind.

4. Biologischer Reaktor nach Anspruch 2, **dadurch gekennzeichnet, dass** die Wände (16) eine krummlinige Abwicklung aufweisen.

5. Biologischer Reaktor nach Anspruch 1, **dadurch gekennzeichnet, dass** die Füllkörper (18) eine spezifische Oberfläche, die zwischen 200 und 500 m2 / m3 umfasst ist, aufweisen.

6. Biologischer Reaktor nach einem der Ansprüche 1 bis 5, **dadurch gekennzeichnet, dass** die Füllkörper (18) eine spezifische Oberfläche von 280 m2 / m3 aufweisen.

7. Biologischer Reaktor nach einem oder mehreren der vorhergehenden Ansprüche, **dadurch gekennzeichnet, dass** das Aufbewahrungsmittel ein Käfig ist, der durch ein Metallnetz (20) definiert ist, dessen Maschen geeignet sind, ein leichtes Austreten von während des Prozesses entwickelten bakteriellen Ausbeutestücken zu ermöglichen.

## Revendications

1. Réacteur biologique (10) du type adhérant ou à biomasse fixe, destiné à épurer des déchets liquides industriels ou civils et/ou à cultiver des micro-organismes pour des processus microbiologiques lors de la préparation de substances chimiques et pharmaceutiques, comprenant :
- un réservoir ou un bassin (14) approprié afin de contenir une substance nutritive ou des eaux résiduaires ;
- un élément de mise en contact biologique (12) immergé partiellement ou entièrement dans ledit réservoir (14) et pouvant tourner par rapport à un axe horizontal (13) ;
- un compresseur (26) et un ou plusieurs diffuseurs (24) agencés sur le fond dudit réservoir (14), adaptés pour produire et souffler un courant d'air ou de gaz afin de faire tourner ledit élément de mise en contact (12) ;
ledit élément de mise en contact (12) comprenant une pluralité de secteurs concaves définis par des parois (16) ayant une forme qui présente un ou plusieurs angles aigus agencés à l'intérieur de l'élément de mise en contact (12), s'étendant longitudinalement de manière continue ou par parties sur la totalité de la longueur de l'élément de mise en contact (12) et des corps de garnissage (18) agencés à l'intérieur desdits secteurs, garnissant la totalité du volume de l'élément de mise en contact biologique (12) et adaptés pour augmenter la surface d'échange entre micro-organismes, gaz et liquide ;
des moyens de confinement enveloppant lesdits secteurs et corps de garnissage (18) et permettant le passage de liquides.

2. Réacteur biologique selon la revendication 1, **caractérisé en ce que** lesdits secteurs définis par des parois (16) se développent radialement à la base en partant de l'axe de rotation (13).

3. Réacteur biologique selon la revendication 1 ou 2, **caractérisé en ce que** lesdites parois (16) sont développées suivant une direction rectiligne et sensiblement radiale et sont formées avec une ou plusieurs courbes afin de définir certains dômes.

4. Réacteur biologique selon la revendication 2, **caractérisé en ce que** lesdites parois (16) présentent un développement curviligne.

5. Réacteur biologique selon la revendication 1, **caractérisé en ce que** les corps de garnissage (18) présentent une surface spécifique comprise entre 200 et 500 m²/m³.

6. Réacteur biologique selon les revendications 1 à 5, **caractérisé en ce que** lesdits corps de garnissage (18) présentent une surface spécifique égale à 280 m²/m³.

7. Réacteur biologique selon une ou plusieurs des revendications précédentes, caractérisé en ce ledit moyen de confinement est une cage définie par un filet métallique (20) dont les mailles sont adaptées pour permettre une sortie facile des déchets bactériens développés au cours du processus.
